# EUROPEAN PATENT APPLICATION

(11) **EP 1 917 924 A1**
(43) Date of publication of application: **07.05.2008**
(21) Application number: 06022837.6
(22) Date of filing: 02.11.2006
(51) Int. Cl.: A61B 17/72

(54) **Implantation device**

(71) Applicant: Stryker Trauma GmbH, 24232 Schönkirchen/Kiel (DE)
(72) Inventor: Schwager, Manuel, 4500 Solothurn (CH); Zander, Nils, 24340 Eckenförde (DE); Henry, Philip, 2503 Biel/Bienne (CH)
(74) Representative: Haeusler, Rüdiger Hans-Jörg

(57) **Abstract**

According to an exemplary embodiment an implantation device comprises an implantation device for implantation in a target structure, the implantation device comprises a shaft region, wherein the shaft region comprises a at least one protrusion, wherein the protrusion comprises a material which is modifiable by using mechanical energy. In particular, the modifiable material may be liquefiable and/or shearable by the mechanical energy. Preferably, the material may be adapted in such a way that the modifications may be achievable by a energy input which does not destroy human tissue.

## Description

### Field of Invention

The invention relates to an implantation device, an implantation kit and a method for applying the same, in particular a bone pin which is formed as a sonic pin.

### Technical Background

In the prior art several implantation devices for humans or animals are known. The implants at least partly create positive-fit connections to human or animal tissue parts, particularly skeletal parts, wherein the implants help connect tissue parts together, or help connect tissue parts to means supporting or replacing tissue parts, or to other therapeutic auxiliary devices. Further, methods for implanting implants into humans or animals are known.

Known implants for creating connections to skeletal parts (bones) include screws, pins, staples, etc., which are used for connecting bones to bones, or bones to artificial, carrying, stabilizing, or supporting parts, or to parts replacing skeletal parts (stabilization or fixation plates, sutures, wires, artificial joint elements, artificial teeth, bone grafts, etc.). Such connection elements for implantation consist for example of metal or plastic, including resorbable plastic. After healing, the connection elements are removed by a further operation S 8096 / KK:RH:ar or they are left in the body where they are possibly gradually decomposed and replaced by vital tissue.

For stabilizing a bone fracture, a fixation plate with suitable holes is fixed in the region of the fracture using screws as mentioned above. Plate and screws may consist of metal (e.g. stainless steel or titanium). The screws are self-cutting and are rotated into thread less openings in the bone, or they are screwed into pre-drilled threaded openings. Pins are pushed into previously created openings for similar purposes. Connections created in the foregoing manner are usually based on frictional engagement, possibly on positive fit.

It is known also to use curable, plastic materials (e.g. particular cements on a hydraulic or polymer base) for creating connections of the mentioned type. Such materials are pressed from the outside between implant and vital tissue, or into tissue defects in a highly viscous condition, and are cured in situ. Positive-fit connections can be created using such material, if the openings into which the material is pressed comprise suitable undercuts. In order to reduce the stress and/or costs of the corresponding operation method so-called biodegradable implants, e.g. bone pins are used. That is, bone pins which degrade over time and which are then absorbed by the body. One of such known biodegradable bone pins is known under the trademark Polypin. This bone pin consists of a polyactid-copolymer mixture and is absorbed during a period of about two years.

Also known in the art is the usage of thermoplastic polymer materials which can be liquefied in a targeted manner by way of mechanical oscillation and, in this condition, can be pressed into cavities by way of hydrostatic pressure, thereby creating positive fit connections after solidification.

Such implants may serve for creating positive-fit connections to tissue parts and may consist at least partly of a material that can be liquefied at a relatively low temperature (<250°C) by way of mechanical oscillation energy, such that the material can be pressed into pores or other openings of the tissue part by the effect of external pressure to form positive-fit connections when re-solidified. For applying the mechanical energy a so-called sonotrode may be used which can be coupled to the implant, e.g. bone pin, and applies the mechanical energy to the bone pin.

For example, from US 6 921 264 an implant for implantation in human or animal bone tissue or in bone tissue supplemented with bone substitute material is known, wherein at least a part of the implant surface comes into contact with the bone tissue, wherein said part of the implant surface comprises surface regions of a first type and surface regions of a second type being different from the surface regions of the first type, wherein the surface regions of the second type comprise a material which is liquefiable by mechanical oscillation and with the aid of which on implantation by mechanical oscillation the implant is stabilized at least primarily in the bone tissue.

### Summary of the Invention

However, there may be a need to provide an implantation device, an implantation kit and a method for implanting an implantation device which implantation device provides for a more efficiently modifying process when applying ultrasonic energy.

This need may be met by an implantation device, an implantation kit and a method for implanting an implantation device according to the independent claims.

According to an exemplary embodiment an implantation device for implantation in a target structure is provided, the implantation device comprises a shaft region, wherein the shaft region comprises a plurality of protrusions, wherein the plurality of protrusions comprise a material which is modifiable by using mechanical energy. Preferably, the plurality of protrusions are arranged on the shaft in such a way that a flow of liquefied material along the shaft is reduced. In particular, the modifiable material may be liquefiable and/or shearable by the mechanical energy. Preferably, the material may be adapted in such a way that the modifications may be achievable by energy input which does not destroy human tissue. For example, the material may be adapted to exhibit the modification at temperatures below a predetermined temperature threshold, wherein the temperature threshold may be below 250°C. Preferably, the material may be a biocompatible material.

According to an exemplary embodiment an implantation kit comprises an implantation device according to an exemplary embodiment of the invention. Preferably, the implantation kit further comprises an implantation device applicator, an ultrasonic device and an implantation device remover.

According to an exemplary embodiment a method for implanting an implantation device according to an exemplary embodiment of the invention comprises implanting the implantation device into a target structure and preferably modifying the implantation device by applying mechanical energy.

The idea of an exemplary embodiment may be seen in that an implantation device is provided comprising a shaft having several protrusions which comprise a material which is modifiable by mechanical energy. That is, an implantation device may be provided which comprises protrusions which project from the substantially smooth shaft region, wherein the shaft region may be circular, elliptical or in the shape of a polygon in cross-section. These protrusions or projections may then be in contact to the surrounding target structure in such a way that when mechanical energy is applied to the implantation device preferably this protrusion is modified, for example liquefied, melted or sheared off. The modified material then may act as a bonding agent to bond the implantation device to the surrounding target structure. Since the protrusions project from the substantially smooth surface of the shaft region of implantation device only a small energy input may be necessary to modify the material of the protrusion. Thus, possibly only a small total input of energy may be necessary in order to bond the implantation device into the target structure, which may lead to the advantage that harmful effects on the surrounding target structure may be omitted or at least reduced. Providing several protrusions may further exhibit the advantage that while the implantation device is implanted into the target structure friction is reduced, since only the protrusions may come in contact with the surrounding target material, which may ease the application of the implantation device. The implantation device may be a bone pin or bone plug used to fix a fracture of a bone, for example a human or animal bone. By providing a plurality of protrusions which are made of the modifiable material it may be possible to improve a bonding between the implantation device and the surrounding target structure. Furthermore, it may be possible to reduce the friction between the implantation device and the surrounding target structure even more while implanting the device.

In the following, further exemplary embodiments of the implantation device will be described. However, these embodiments apply also for the implantation kit and the method for applying the implantation device.

According to another exemplary embodiment of the implantation device the mechanical energy is ultrasonic energy.

The use of ultrasonic energy may be an efficient way to provide mechanical energy sufficient to modify the material of the protrusions. In particular, ultrasonic energy may be suitable to omit harmful effects in the surrounding target structure. Furthermore, the use of ultrasonic waves may be suitable to shear off small parts of the modifiable material of the protrusions, which small parts behave substantially like a liquid, so that it may be said that the material is liquefied. When the input of ultrasonic energy is switched off the liquefied material may solidify again so that a strong bonding between the implantation device and the surrounding target structure may be achievable. A suitable frequency of the used ultrasonic energy may be in the range between 28 kHz and 31 kHz. In particular, the frequency may be 29.5 kHz. A suitable amplitude may be in the range between 15 µm and 25 µm. In particular, the amplitude may be about 20 µm.

According to another exemplary embodiment of the implantation device the modifiable material is bioabsorbable. That is, the material may be absorbed by a human or animal body. Preferably, the bioabsorbable material comprises a copolymer comprising between 50% and 90% Poly-L-lactide and between 10% and 50% Poly-D, L-lactide. In particular, the bioabsorbable material may be a copolymer comprising 70 weight% Poly-L-lactide and 30 weigh% Poly-D, L-lactide. Preferably, the bioabsorbable material may be formed as an amorphous material.

The above described material may be a suitable material for an implantation device, which material may exhibit a suitable tensile strength of about 60 MPa, and a suitable E-modulus of about 3500 MPa. Furthermore, the above material, i.e. an implantation device comprising the above material, may retain its strength for about a sufficient time when implanted into a human or animal's body. Such a time span may be about 16 to 26 weeks. The described copolymer may have a resorption time of about two to three years in a human or animal body. The material may further exhibit an increase of implant volume up to 200% after 24 month from the implantation in the target structure. Such a material may further be easily to be sterilized by γ-radiation. A suitable energy dose may be between 20 kGy and 30 kGy, in particular below 25 kGy. According to another exemplary embodiment the whole implantation device may comprise or may be formed by the bioabsorbable material.

By forming the whole implantation device by the bioabsorbable material the use of the implantation device may be simplified. In particular, no additional operation may be necessary to remove the implantation device or parts of it after a healing process of the target structure has been completed.

According to another exemplary embodiment of the implantation device the shaft has a substantially longitudinal shape and preferably the plurality of the protrusions are arranged displaced, staggered or misaligned to each other along a longitudinal axis of the longitudinal shape. In particular, the plurality of protrusions may be arranged in a pattern which looks like a chess-board pattern, i.e. in a first row of protrusions wherein the protrusion are spaced from each other, while in a next row of protrusions the individual protrusions are placed at the positions of the spaces of the previous row. By arranging the protrusions in such a manner a pattern may be introduced which hinders a flow of the modified or liquefied material along the shaft of the implantation device, i.e. the rows of protrusions may form a barrier hindering the flow. By forming such a barrier it may be possible to prevent that the liquefied material flows out which may lead to the fact that the bonding may be more secure due to the fact that more liquefied material may solidify after the input of mechanical energy is switched off.

According to another exemplary embodiment of the implantation device the protrusions having a polygonal shape. For example, the protrusions may have a shape of a triangle, a square, trapezoid, or a rectangle. Also circular or elliptical shapes may be suitable. Alternative shapes may be elongated protrusions or tongues, i.e. along the circumference or along the longitudinal axis of the shaft or perpendicular to the longitudinal axis. But also irregular protrusions may be possible. The choosing of the shape may be performed depending on the size of the protrusions and on the possibility to hinder the out flow of liquefied material. A further criterion for the shape may be the ease to shear off parts of the protrusions. In particular, the protrusion may have the polygonal shape in a cross sectional view of the shaft region. Along specific sections of the shaft the cross-section may be identical, but may be different to other, e.g. consecutive sections of the shaft regions. In a side view of the shaft region the individual sections or segments may have a polygonal shape as described above.

The different segments, i.e. one segment arranged between two other segments of different cross section, may form channel regions in the shaft region, which channel region may facilitate a main flow of liquefied material in a direction substantially perpendicular to the longitudinal axis of the shaft region, while hindering a flow along the longitudinal.

According to another exemplary embodiment of the implantation device the implantation device has a colour which is clearly distinguishable from a colour of the target structure. In particular, the protrusions may have blue colour, which may enable a good visibility of the implantation device when the same is implemented into a human or animal body. Preferably, the bone pin is coloured by mixing colour of pigment into the mass the implantation device is made from, e.g. a polymer. Alternatively, the surface of the formed implantation device can be painted by the colour.

According to another exemplary embodiment of the implantation device the plurality of protrusions are formed in such a way that during implanting the implantation device into a target material only few protrusions are in contact with the target material, i.e. that not the whole shaft is in contact with the target structure. In particular, the plurality of protrusions may be formed in such a way that during an application of the mechanical energy only a few protrusions are in contact with the target structure. This may lead to the advantage that only at these contact points energy is used to modify, e.g. liquefy, the material so that the total power which may be inputted into the implantation device may be reduced.

In the following, a further exemplary embodiment of the method will be described. However, this embodiment apply also for the implantation device and for the implantation kit.

According to another exemplary embodiment of the method the modifying is partially done by shearing of parts of the implanted implantation device. That is, the modifying, is not only performed by melting the material, e.g. by friction, but at least a part of the modifying is performed by shearing off or grating small parts of the material.

It may be seen as the gist of an exemplary embodiment to provide a structure for an implantation device, e.g. a bone pin, which allows to minimize an energy input into the implantation device and thus to a target structure, while still allowing to modifying a state of a material of the implantation device. In order to achieve this, the implantation device may comprise a plurality of protrusions projecting from a substantially smooth surface of a shaft of the implantation device. When implanting and fixing this implantation device into the target structure, e.g. broken bone which shall be fixated by the implantation device, mechanical energy like ultrasonic energy is applied to the implantation device leading to a modifying or liquefying of the material of at least the protrusion which liquefied material might be used as a bonding agent promoting a bonding force between the implantation device and the target structure. Due to the small size of the protrusion a contact area between the implantation device and the target structure may be minimized leading to a relatively high energy per area so that grating or melting of the protrusion is promoted although only relatively low energy input and power, i.e. energy per time, is necessary. The implantation device, e.g. a bone pine, according to an exemplary embodiment of the invention may have a surface which is rougher than the surface of known bone pins. This roughness may be caused by the plurality of protrusions which may also be called energy raisers. For testing two different types of bone pins, i.e. one having a rough surface according to an exemplary embodiment of the invention while the other one having a smooth surface as known in the prior art, are inserted in bone substitute material. The energy input needed to insert the pins to the same depth was measured. In the testing a clear difference in energy input needed to insert the bone pin arose. For a bone pin according to an exemplary embodiment of the invention, i.e. a rough bone pin, about 19% less energy input is needed when compared to a known, i.e. smooth bone pin. In particular, the mean energy input needed for the known bone pin was about 58.6 J, while for a rough bone pin, i.e. a bone pin having a plurality of protrusion, the mean energy input was about 47.3 J. Also the fusion time was reduced from about 2.5 s to about 2 s in the case of a bone pin according to an exemplary embodiment of the invention was used.

According to an exemplary aspect a bone pin may be made of resorbable material, e.g. plastic or polymer, which bone pin features the characteristics that it melts at well defined positions when ultrasound is applied to it. The liquefied polymer may spread out preferably perpendicular to the axis of the bone pin, while spreading out parallel to the axis may be suppressed by fluid barriers which may be formed by protrusions projecting from the surface. Furthermore, a moment of inertia of the bone pin may be substantially equal or at least may not show a great variation along its axis, which may be made possible by a plurality of polygonal protrusions, e.g. in the shape of triangles, which are misaligned relative to each other and which may form a flow barrier. The ultrasonic energy may be concentrated on this certain points on the surface due to the steps formed by the protrusions, e.g. the triangles.

According to the present invention also implantation devices comprising a metal, e.g. titanium, core may be used which have a polymeric overlay, i.e. an overlay comprising a modifiable material. Also bone screws may be provided, in particular bone screws having a metal portion and a tip comprising modifiable material, wherein at the portion of the modifiable material protrusions are formed.

Implantation devices according to an exemplary embodiment may be used for plate fixation in which application the implantation devices, e.g. resorbable pins may be welded to bone and resorbable plate. The use of resorbable pins may provide a fast fixation and a better stability. A further application may be hip fracture fixation. In this application a metal bone screw having a tip of polymeric material, i.e. modifiable material comprising protrusion, may be used. Also this kind of implantation devices may exhibit fast fixation. Further, it might be possible that no rotation of the implantation device is necessary, which rotation possible would displace fragments, due to the fact that the implantation device, e.g. the shaft, does not comprise a thread. Furthermore, such a bone screw having a polymeric tip may be exhibit a better resistance to cut-off.

In general, implantation devices according to an exemplary embodiment may be used in different fields of fixation of bone fragments. In particular, resorbable bone pins may be used in the field of small fragment fixation of foot, ankle, wrist, elbow and shoulder, resorbable mesh/plate and pins may be used in the field of small fragment fixation of ankle, wrist, and graft containment and hybrid metal and resorbable pins may be used in the field of distal locking, bone anchor, femoral neck fractures, trochaneric fractures, and ex fix pin fixation in osteoporotic bones.

These and other aspects of the present invention will become apparent from and elucidated with reference to the embodiment described hereinafter.

### Brief Description of the Drawings

An exemplary embodiment of the present invention will be described in the following, with reference to the following drawings.
Fig. 1A shows a schematic illustration of a bone pin according to an exemplary embodiment; and
Figs. 1B to 1I are showing enlarged detailed views of the bone pin of Fig. 1A.
Fig. 2 shows a schematic illustration of an ultrasonic device;
Fig. 3 show schematic illustrations of an implantation device applicator according to an exemplary embodiment of the invention; and
Fig. 4 show schematic illustration of an implantation device remover according to an exemplary embodiment of the invention.

### Detailed Description of an Exemplary Embodiment

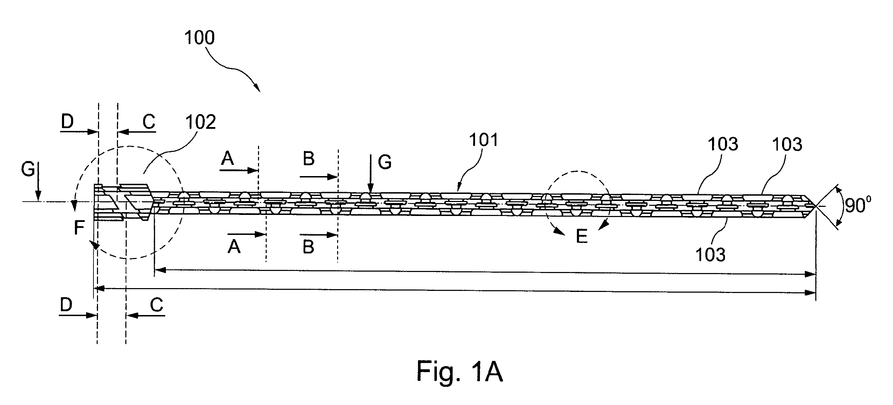

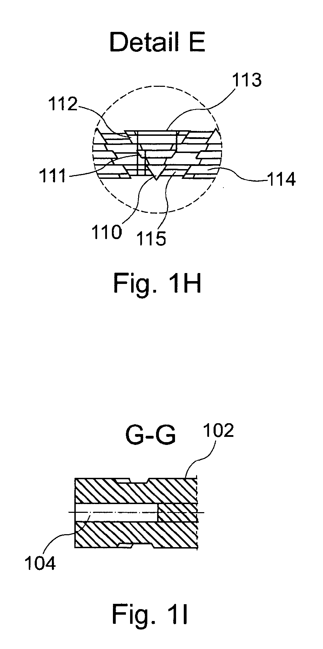

The illustration in the drawings is schematically. In different drawings, similar or identical elements are provided with similar or identical reference signs.Fig. 1A shows a schematic illustration of the bone pin 100 according to an exemplary embodiment. The bone pin 100 comprises a shaft region 101 and a base region 102. The shaft region 101 comprises a plurality of protrusions 103 which roughly have the general shape of a triangle. A single one of the protrusions is depicted in greater detail in Fig. 1H. The base region 102 may be adapted to fit onto a corresponding ultrasonic device.

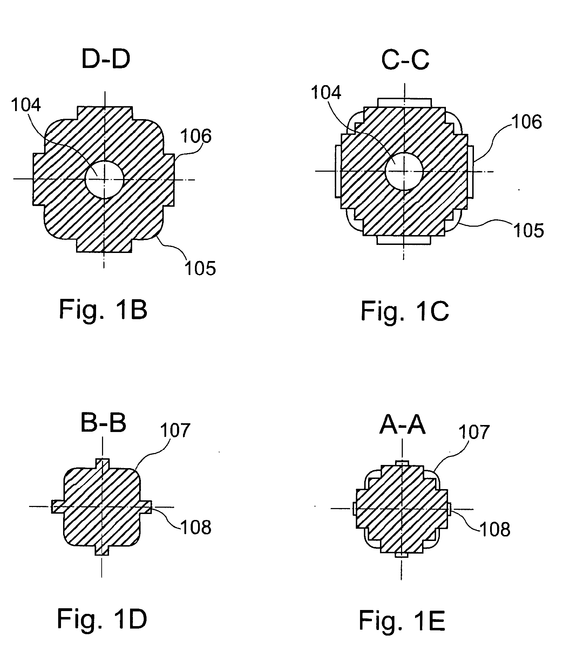

Fig. 1B shows an enlarged view of the sonic pin 100 of Fig. 1A. In particular, it shows a cross section along line D-D of Fig. 1A, i.e. a cross section of the base region 102. In the centre of the cross section of Fig. 1B a hole 104 is shown which might be adapted to accommodate a portion of the ultrasonic device, e.g. a sonotrode of the ultrasonic device. Furthermore, it can be seen in Fig. 1B that the cross section comprises rounded regions 105 and substantially plane regions 106.

Fig. 1C shows another enlarged view of the sonic pin 100 of Fig. 1A. In particular, it shows a cross section along line C-C of Fig. 1A, i.e. also a cross section of the base region 102. In the centre of the cross section of Fig. 1C the hole 104 is shown. Furthermore, in Fig. 1C the rounded regions 105 and the substantially plane regions 106 can be seen.

Fig. 1D shows another enlarged view of the sonic pin 100 of Fig. 1A. In particular, it shows a cross section along line B-B of Fig. 1A, i.e. a cross section of the shaft region 101. Also this cross section shows further rounded regions 107 and further substantially plane regions 108. In particular, the further plane regions 108 may be part of the protrusions 103.

Fig. 1E shows another enlarged view of the sonic pin 100 of Fig. 1A. In particular, it shows a cross section along line A-A of Fig. 1A, i.e. a cross section of the shaft region 101. Also this cross section shows the further rounded regions 107 and the further substantially plane regions 108.

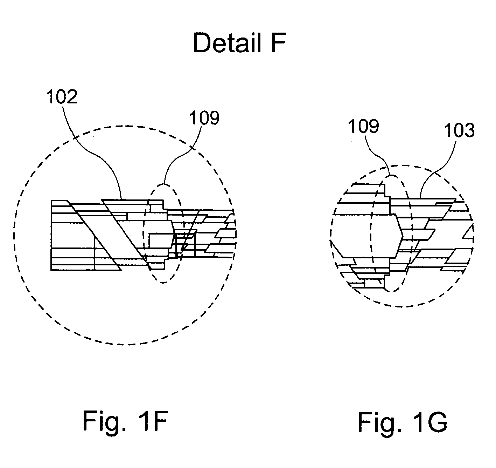

Fig. 1F shows another enlarged view of the sonic pin 100 of Fig. 1A. In particular, it shows a detailed view of the portion which corresponds to the circle labeled F in Fig. 1A, i.e. of the base region 102. In particular, Fig. 1F shows the transition portion 109 between the base region and the shaft region.

Fig. 1G shows an even more enlarged view of the portion of Fig. 1F, in which the transition region can be seen more clearly. In particular Fig. 1G also shows a part of a protrusion 103 in more detail. The protrusions of the embodiment shown in Fig. 1 have an overall shape, which is roughly triangular. The single triangles are formed by tongues 110, 110 and 112 which do have a different length along the axis of the sonic pin 100. Due to the different lengths a roughly triangular shape of the protrusions results. Such a shape may be suitable to hinder the flowing of liquefied material along the axis of the sonic pin 100 and promote a more perpendicular spreading of the liquefied material, i.e. a spreading which promote the flowing of the liquefied material into cavities or pores which are formed in bone material of human or animal bones.

Fig. 1H shows another enlarged view of the sonic pin 100 of Fig. 1A. In particular, it shows a detailed view of the portion which corresponds to the circle labeled E in Fig. 1A, i.e. a protrusion formed in the shaft region 101. As well as in Fig. 1 G the roughly triangular shape of the protrusion can be seen in Fig. 1H, which shape is formed by the tongues 110, 111, and 112 having different lengths. Furthermore, it can be seen in Fig. 1H that the orientation of the triangular protrusion 103 are altering, i.e. after a first triangular protrusion 113 having its base length in Fig. 1H at the upper side, a consecutive triangular protrusion 114 has its base length at the bottom side, leading to channel regions 115 between the single triangles, which channels 115 may be suitable to direct the flow of liquefied material.

Fig. 1I shows another enlarged view of the sonic pin 100 of Fig. 1A. In particular, it shows a detailed cross sectional view along line G-G in Fig. 1A, i.e. a cross sectional view of the base region 102. In particular, the hole 104 in the base region can be seen, in which a tip of a sonotrode or an implantation device applicator may be inserted.

A bone pin according to an exemplary embodiment may be used in a operation process for fixation of a broken or splintered bone, the procedure comprising the following steps. Drilling a hole in which the bone which has to be fixed afterwards the bone pin is implemented into the drilled hole. In case the bone is a porous bone, i.e. comprising porous material it may not necessary to pre drill a hole. Preferably, this is done using an so-called pin applicator. Afterwards the pin applicator is removed and an ultrasonic device is arranged at the base region of the bone pin, i.e. in the hole which is shown as 104 in Fig. 1. When the ultrasonic device is switched on the bone pin starts to oscillate and the protrusions arranged on the shaft of the bone pin come into contact with the surrounding bone so that portions of the protrusions are sheared off by shearing forces from the bone pin, whereby they are liquefied. Due to the providing of a plurality of protrusion comprising tongues only small parts of the hole shaft of the bone pin are contacting the bone and are sheared off or liquefied. The liquefied material of the bone pins starts to penetrate pore spaces of the bone. When the ultrasonic device or ultrasonic driver is switched off the liquefied material, e.g. the polymer, cools rapidly, resulting in a stable joint after only a few seconds and may form a positive locking. In case the bone pin should be removed again a bone pin remover may be used. Such a pin remover may comprise a portion similar a crown drill, which drill is placed above the bone pin which is to be removed. Then the remover is drilled into the bone. The cutting hull or cutting sheath of the crown drill cuts into the bone and also cuts a thread into the bone cut in the cutting hull. This threading may provide a positive locking between the driller and the bone portion to be removed so that afterwards the remover may be used to break the bone portion including the bone pin to be removed.

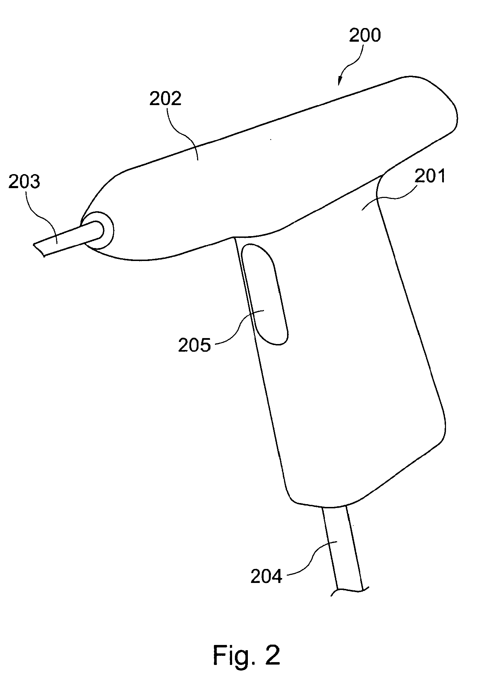

Fig. 2 shows a schematic illustration of an ultrasonic device 200 which can be used in combination with a bone pin according to an exemplary embodiment of the invention. The ultrasonic device comprises a hand piece 201 which has a shape which is adapted to be gripped by a human, e.g. a physician implementing the bone pin. Furthermore, the ultrasonic device comprises a mounting part 202 in which a sonotrode 203 can be inserted. Furthermore, a cable 204 for supplying the ultrasonic device with energy and a switch 205 for turning the ultrasonic device on and off is shown in Fig. 2.

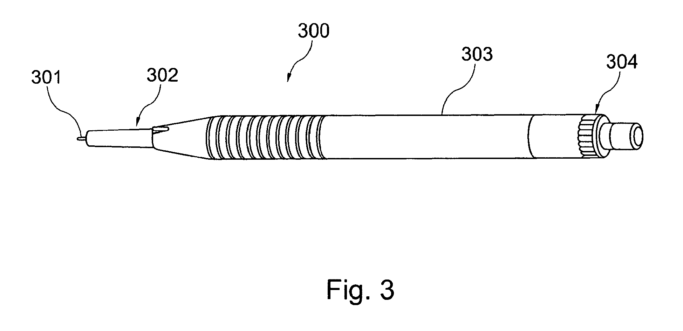

Fig. 3 shows schematic illustrations of an implantation device applicator 300 according to an exemplary embodiment of the invention. Fig. 3 shows a perspective view of the implantation device applicator 300. The implantation device applicator 300 comprises a tip 301 and a front region 302. Furthermore, the implantation device applicator 300 comprises a main body 303 and an end region 304.

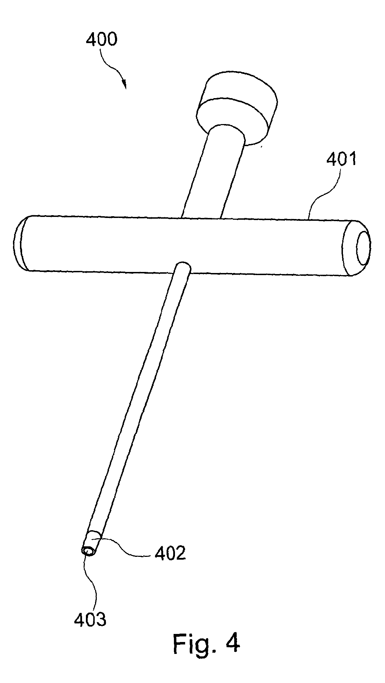

Fig. 4 shows a schematic view of an implantation device remover 400 according to an exemplary embodiment. The implantation device remover 400 comprises a handle 401, which is in the case of Fig. 4 a simple T-piece. The handle 401 serves for a good grip of a person using the implantation device remover, e.g. a surgeon. The implantation device remover 400 further comprises a drilling region 402 which comprises at a tip of the drilling region 402 a hollow part. Furthermore, the tip comprises an edge 403 which is formed like a saw, e.g. comprises cutting teeth. In total the implantation device remover 400 has a size which is suitable for good gripping by the surgeon, e.g. the handle is about 100 mm wide. The diameter of the drilling region 402 is adapted to the diameter of the implantation device which is to be removed. In particular, the inner diameter of the hollow part may be a little greater than the diameter of the implantation device, e.g. a bone pin.

Summarizing it may be seen as one aspect of the present invention to provide a bone pin having a shaft comprising a plurality of protrusions. Due to the protrusions only small parts of the bone pins are sheared off when ultrasonic energy is applied to the bone pin, leading to the effect that only a small total power is used to achieve a suitable energy density at the protrusions to liquefy the same. Thus, the total power, i.e. energy per time, may be reduced which may reduce the energy input into the bone the bone pin is applied to, so that damage to the bone may be reduced.

It should be noted that the term "comprising" does not exclude other elements or steps and the "a" or "an" does not exclude a plurality. Also elements described in association with different embodiments and aspects may be combined. It should also be noted that reference signs in the claims shall not be construed as limiting the scope of the claims.

## Claims

1. An implantation device for implantation in a target structure, the implantation device comprising:
a shaft region,
wherein the shaft region comprises a plurality of protrusions;
wherein the plurality of protrusions comprises a material which is modifiable by using mechanical energy;
wherein the plurality of protrusions are arranged on the shaft region in such a way that a flow of liquefied material along the shaft is reduced..

2. The implantation device according claim 1,
wherein the mechanical energy is ultrasonic energy.

3. The implantation device according to claim 1 or 2,
wherein the modifiable material is bioabsorbable.

4. The implantation device according to claim 3,
wherein the whole implantation device may comprise the bioabsorbable material.

5. The implantation device according claim 3 or 4,
wherein the bioabsorbable material comprises a copolymer comprising between 50% and 90% Poly-L-lactide and between 10% and 50% Poly-D, L-lactide.

6. The implantation device according to anyone of the preceding claims,
wherein the shaft has a substantially longitudinal shape.

7. The implantation device according to claim 6,
wherein the plurality of the protrusions are staggered to each other along a longitudinal axis of the longitudinal shape.

8. The implantation device according to anyone of the preceding claims,
wherein the protrusions having a polygonal shape.

9. The implantation device according to anyone of the preceding claims, wherein the implantation device has a colour which is clearly distinguishable from a colour of the target structure.

10. The implantation device according to anyone of the preceding claims,wherein the plurality of protrusions are formed in such a way that during implanting the implantation device into a target material only few protrusions are in contact with the target material.

11. An implantation kit comprising:
an implantation device according to anyone of the claims 1 to 10.

12. The implantation kit according claim 11, further comprising:
an implantation device applicator;
an ultrasonic device;
an implantation device remover.

13. A method for implanting an implantation device according to anyone of the claims 1 to 10, the method comprising:
implanting the implantation device into a target structure; and
modifying the implantation device by applying mechanical energy.

14. The method according to claim 13,
wherein the modifying is partially done by shearing of parts of the implanted implantation device.

## Amended claims

### Amended claims in accordance with Rule 137(2) EPC.

**1.** An implantation device for implantation in a target structure, the implantation device comprising:
a shaft region,
wherein the shaft region comprises a plurality of protrusions;
wherein the plurality of protrusions comprises a material which is modifiable by using mechanical energy;
wherein the plurality of protrusions are arranged on the shaft region in such a way that a flow of liquefied material along the shaft is reduced..

**2.** The implantation device according claim 1,
wherein the mechanical energy is ultrasonic energy.

**3.** The implantation device according to claim 1 or 2,
wherein the modifiable material is bioabsorbable.

**4.** The implantation device according to claim 3,
wherein the whole implantation device may comprise the bioabsorbable material.

**5.** The implantation device according claim 3 or 4,
wherein the bioabsorbable material comprises a copolymer comprising between 50% and 90% Poly-L-lactide and between 10% and 50% Poly-D, L-lactide.

**6.** The implantation device according to anyone of the preceding claims,
wherein the shaft has a substantially longitudinal shape.

**7.** The implantation device according to claim 6,
wherein the plurality of the protrusions are staggered to each other along a longitudinal axis of the longitudinal shape.

**8.** The implantation device according to anyone of the preceding claims,
wherein the protrusions having a polygonal shape.

**9.** The implantation device according to anyone of the preceding claims,
wherein the implantation device has a colour which is clearly distinguishable from a colour of the target structure.

**10.** The implantation device according to anyone of the preceding claims,
wherein the plurality of protrusions are formed in such a way that during implanting the implantation device into a target material only few protrusions are in contact with the target material.

**11.** An implantation kit comprising:
an implantation device according to anyone of the claims 1 to 10.

**12.** The implantation kit according claim 11, further comprising:
an implantation device applicator;
an ultrasonic device;
an implantation device remover.

**13.** A method for handling an implantation device according to anyone of the claims 1 to 10, the method comprising:
implanting the implantation device into a target structure; and
modifying the implantation device by applying mechanical energy.

**14.** The method according to claim 13,
wherein the modifying is partially done by shearing of parts of the implanted implantation device.
